Europäisches Patentamt

European Patent Office

Office européen des brevets

(11) Publication number: **0 331 385 B1**

## EUROPEAN PATENT SPECIFICATION

(45) Date of publication of patent specification: **30.03.94**　(51) Int. Cl.⁵: **A61K 31/70**, A61K 47/36

(21) Application number: **89301904.2**

(22) Date of filing: **27.02.89**

The file contains technical information submitted after the application was filed and not included in this specification

(54) **Aqueous suspension of sucralfate.**

(30) Priority: **02.03.88 JP 47423/88**

(43) Date of publication of application:
**06.09.89 Bulletin 89/36**

(45) Publication of the grant of the patent:
**30.03.94 Bulletin 94/13**

(84) Designated Contracting States:
**AT BE CH DE FR GB IT LI NL SE**

(56) References cited:
**EP-A- 0 136 100　　EP-A- 0 245 855**
**EP-A- 0 286 978　　WO-A-86/01406**
**DE-A- 3 322 078　　FR-A- 2 598 084**

**PATENT ABSTRACTS OF JAPAN vol. 8, no. 52 (C-213)(1489) 9 March 1984**

(73) Proprietor: **Chugai Pharmaceutical Co., Ltd.**
**5-1, Ukima 5-chome**
**Kita-ku**
**Tokyo 115(JP)**

Proprietor: **SATO PHARMACEUTICAL CO., LTD.**
**8-5, Higashi-ohi 6-chome**
**Shinagawa-ku**
**Tokyo(JP)**

(72) Inventor: **Katayama Masahide c/o Sato Pharmaceutical Co Ltd**
**8-5, Higashi-ohi 6-chome**
**Shinagawa-ku Tokyo(JP)**
Inventor: **Yamashita Harushige c/o Sato Pharmaceutical Co.Ltd**
**8-5, Higashi-ohi 6-chome**
**Shinagawa-ku Tokyo(JP)**
Inventor: **Odaka Taizo c/o Sato Pharmaceutical Co. Ltd.**
**8-5, Higashi-ohi 6-chome**
**Shinagawa-ku Tokyo(JP)**
Inventor: **Morioka Shigeo c/o Sato Pharmaceutical Co. Ltd.**
**8-5, Higashi-ohi 6-chome**
**Shinagawa-ku Tokyo(JP)**

(74) Representative: **Ouest, Barry et al**
**M'CAW & Co.**
**41-51 Royal Exchange**
**Cross Street**
**Manchester M2 7BD (GB)**

**Description**

The invention relates to a suspension containing sucralfate (sucrose sulfate aluminum salt) as pharmaceutically effective component.

The sucralfate can combine directly with pepsin in gastric juice to suppress the activity thereof. It has antipepsin and antiacid effects. The sucralfate can form a covering film over mucous membranes of stomach and duodenum to protect the membrane. This effect is to selectively couple with ulcer area of the digestive tract for the protection of the diseased part.

Pharmaceutical preparations containing this sucralfate have been provided in the solid form such as tablets, granules, pellets or powders. Since the sucralfate is not water soluble, the liquid form of the preparation must be suspension but any of the usuall assitants can not hold the suspension stable for a long time.

JP-A 503031/1986 discloses a suspension containing sucralfate, to which 1 - 5 weight % of xanthane gum and 1 - 12.5 weight % of at least one peptizer are added. This is not satisfactory, however, in that it is necessary to control the particle dimension of sucralfate to be smaller than 50 $\mu$m since if the particle dimension is larger than that the suspension is unstable due to particle sedimentation. Since the amount referred to above of xanthane gum to be added is in relation to sucralfate, viscosity of the suspension is too low when sucralfate content therein is fairly small. It is necessary in this case to add glycerin or the like in order to increase the viscosity. In case where sucralfate is added in a considerable amount in the suspension, this is to be a slurry rather than the suspension so as to be difficult to take.

And EP-A-0245855 discloses a sucralfate preparation in the form of suspension having a viscosity of from 1,000 to 5,000 centipoise at 20° C. It is used in the treatment of inflammations of esophageal mucosa, and the preparation comprises water-insoluble polymeric substances such as crystalline cellulose, tragacanth gum, arginic acid, starch and carboxymethylcellulose, water-soluble polymeric substances such as methylcellulose, hydroxyethylcellulose, hydroxyproprylmethylcellulose, carboxymethylstarch, xanthan gum and carggeenane. In order to ensure that sucralfate effectively adheres to inflammatory sites, it is necessary to adjust a viscosity with water-insoluble and water-soluble polymeric substances.

However in the invention of EP-A-0245855 the viscosity of the suspension is high and the redispersibility of the suspension is not satisfactory.

Meanwhile, it is well known that solid pharmaceutical preparations generally require a considerable period of time until the preparations are disintegrated and consequently until the medicinal effect appears, from liquid pharmaceutical preparations.

In order to prepare an aqueous suspension, it is well known to generally use a dispersing agent such as acecia, carboxy methyl cellulose sodium, hydroxypropyl methyl cellulose, hydroxyethyl cellulose, crystalline cellulose, alginate, gelatine and bentonite.

Since sucralfate produces $Al^{3+}$ ions in an aqueous medium so as to cause mutual reaction with such dispersing agents, sucralfate particles are bound together to cause rapid sedimentation, whereby the suspension can not be redispersed or is changed to be in semi-solid state due to gelation of the dispersing agent under influence of such ions.

There is a possibility depending on the kind of the dispersing agents that the dispersed phase tends to cause sedimentation gradually from particles of larger dimension and higher density. In the course of a so-called free sedimentation for a long time, aggromeration is formed in the suspension (caking phenomenon) so that even by strong shaking it is impossible to redisperse it. It is necessary for the suspension that not only sedimentation of the dispersed phase is retarded as far as possible but also redispersion can be readily made.

It is an object of the invention, thus, to provide a stable and readily dispersible aqueous suspension of sucralfate.

It is another object is to manufacture a pharmaceutical preparation in the form of the aqueous suspension referred to above readily and without any considerably much additional expense.

The other objects of the invention and advantages attained thereby are appreciated by those skilled in the art when studying following explanation of the invention in more detail.

The inventors have devoted themselves to various experiments to discover that in an aqueous suspension of sucralfate using hydroxyethyl or hydroxypropyl a slight gel construction of three dimensions was formed in the suspension so that, from the two points of keeping the dispersion particle suspended in the gel network construction and having thixotropy therein, redispersion was easily obtained, in the case of the precipitation of the dispersion particle, by shaking the suspension and that when further adding at least one selected from cellulose derivatives, polysaccharide gums, alginic acid salts and bentonitite, a more preferable aqueous suspension can be obtained, which is stable for a long time even if it contains sucralfate

2

particles each of which is in the order of 100 $\mu$m.

The dispersing agent to be used in the invention is hydroxyethyl or hydroxypropyl starch.

The cellulose derivative added in the suspension in addition to the said starch derivative is a cellulose ether such as methyl cellulose, ethyl cellulose, benzyl cellulose, carboxylmethyl cellulose, carboxylethyl cellulose, sodium carboxymethyl cellulose, hydroxyethyl cellulose, hydroxypropyl cellulose and hydroxypropylmethyl cellulose; a polysaccharide gum such as acacia, tragacanth gum, karaya gum, guar gum, quince seed, carrageenan, locust bean gum, tamarind gum and dextran; or an alginic acid salt such as alginic acid, sodium alginate and propylene glycol alginate.

In relation to the total amount of the sucralfate suspension, generally added are sucralfate in the amount of 0.1 - 50 w/v %, preferably 0.5 - 20 w/v %, the starch and/or the starch derivative in the amount of 0.05 - 10.0 w/v %, preferably 0.1 - 5.0 w/v %, and the further additive(s) such as the cellulose derivatives, polysaccharide gums and bentonite in the amount of 0.05 - 5.0 w/v %, preferably 0.1 - 2.0 w/v %.

The sucralfate suspension according to the invention may comprise, as occasion demands, further an adsorbent such as kaolin and natural aluminum silicate; an antiseptic such as sodium benzoate, ethyl parahydroxybenzoate, methyl para-hydroxybenzoate, propyl para-hydroxybenzoate, butyl para-hydroxybenzoate, dehydroacetic acid and salt thereof; a defoaming agent such as silicone resin; a flavour and a colouring agent such as purified sucrose, glucose and D-sorbitol; an inorganic antiacid such as magnecium aluminosilicate, synthetic aluminum silicate, dried aluminum hydroxide gel and synthetic hydrotalcite; an amino acid such as aminoacetic acid and dihydroxyaluminum aminoacetate; a stomachic crude drug such as aloe, cinamon park, magnolia bark, ginger and ginseng; a cholagogous such as ursodeoxycholic, dehydrocholic acid and powdered bile; an intestinal ordering crude such as geranium herb; an analgesic anodyne and antispamodic such as corydalis tuber and glycyrrhiza; a covering agent such as precipitated calcium carbonate and clacium hydrogenphosphate.

The invention will be explained in more detail in reference to some Examples. It is noted that the invention is not restricted thereto.

Example 1

Prescription;

| | |
|---|---|
| Sucralfate | 1.5 g |
| Magnesium aluminometasilicate | 1.5 g |
| Maize starch | 0.5 g |
| Hydroxypropyl starch | 3.0 g |
| Purified sucrose | 10.0 g |
| Sodium benzoate | 0.05 g |
| Disodium edetate | 0.01 g |
| Ethanol | 1.0 g |
| Flavour | very small quantity |
| Purified water added to be totally | 100.0 ml |

Firstly sucralfate and magnesium aluminometasilicate are weighed and added with 30 ml pure water to be stirred until homogeneous phase is casued. Separately maize starch and hydroxypropyl starch are weighed, and added with 40 ml purified water to be stirred with heating. Said two mixtures are vigorously stirred to be homegenous, to which purified sucrose, sodium benzoate, disodium edetate, ethanol and flavour are added to be dissolved and further added with purified water so as to make the total amount to be 100 ml.

In following Examples 2 - 4, the aqueous suspensions were prepared similar to the above.

Example 2

| Sucralfate | 2.0 g |
|---|---|
| Hydroxypropyl starch | 3.0 g |
| Hydroxypropyl cellulose | 1.0 g |
| Purified sucrose | 10.0 g |
| Sodium benzoate | 0.05 g |
| Purified water added to be totally | 100.0 ml |

Example 4

| Sucralfate | 4.0 g |
|---|---|
| Hydroxypropyl starch | 1.5 g |
| Propylene glycol alginate | 0.3 g |
| Purified sucrose | 10.0 g |
| Sodium benzoate | 0.05 g |
| Purified water added to be totally | 100.0 ml |

Comparative Example 1

| Sucralfate | 1.0 g |
|---|---|
| Hydroxypropyl cellulose | 1.0 g |
| Purified sucrose | 10.0 g |
| Sodium benzoate | 0.05 g |
| Puifiede water added to be totally | 100.0 ml |

Comparative Example 2

| Sucralfate | 2.0 g |
|---|---|
| Carrageenan | 1.0 g |
| Purified sucrose | 10.0 g |
| Sodium benzoate | 0.05 g |
| Purified water added to be totally | 100.0 ml |

Comparative Example 3

| Sucralfate | 2.0 g |
|---|---|
| Sodium carboxymethyl cellulose | 1.0 g |
| Purified sucrose | 10.0 g |
| Sodium benzoate | 0.05 g |
| Purified water added to be totally | 100.0 ml |

Comparative Example 4

| Sucralfate | 1.0 g |
|---|---|
| Xanthane gum | 0.02 g |
| $NaH_2PO_4$ | 0.03 g |
| Purified sucrose | 10.0 g |
| Sodium benzoate | 0.05 g |
| Purified water added to be totally | 100.0 ml |

Preparations of Examples 1, 3 and 5 as well as Comparative Examples 1 - 4 were stored at temperatures of 3°C, 20°C and 40°C respectively for 3 months to examine the stability, results of which are as follows;

| | Immediately After Preparation | After 3 Months (At 3°C) | After 3 Months (At 20°C) | After 3 Months (At 40°C) |
|---|---|---|---|---|
| Example 1 | Stable | Stable | Stable | Stable |
| Example 3 | Stable | Stable | Stable | Stable |
| Example 5 | Stable | Stable | Stable | Slight Sedimentation |
| Comparative Example 1 | Stable | Not Stable (Caking) | Not Stable (Caking) | Not Stable (Caking) |
| Comparative Example 2 | Stable | Not Stable (Gelation) | Not Stable (Gelation) | Not Stable (Gelation and Caking) |
| Comparative Example 3 | Not Stable (Gelation) | Not Stable (Gelation) | Not Stable (Gelation) | Not Stable (Gelation) |
| Comparative Example 4 | Stable | Not Stable (Caking) | Not Stable (Caking) | Not Stable (Caking) |

## Claims

1. Aqueous suspension of sucralfate, into which hydroxyethyl starch or hydroxypropyl starch is added.

6

2. The aqueous suspension as set forth in Claim 1, which is added further with bentonite.

3. The aqueous suspension as set forth in Claim 1 or Claim 2, which comprises sucralfate in an amount of 0.1-50.0 w/v%, preferably 0.5-20.0 w/v%, hydroxyethyl or hydroxypropyl starch in the amount of 0.05-10.0 w/v%, preferably 0.1-5.0 w/v%, and bentonite in amount of 0.05-5.0 w/v%, preferably 0.1-2.0 w/v%.

**Patentansprüche**

1. Wässrige Suspension von Sucralfat, der Hydroxyethylstärke oder Hydroxypropylstärke zugesetzt worden ist.

2. Die wässrige Suspension nach Anspruch 1, der weiterhin Bentonit zugesetzt worden ist.

3. Die wässrige Suspension nach Anspruch 1 oder 2, die Sucralfat in einer Menge von 0,1 - 50,0% (w/v), vorzugsweise 0,5 - 20% (w/v), Hydroxyethylstärke oder Hydroxypropylstärke in einer Menge von 0,05 - 10,0% (w/v), vorzugsweise 0,1 - 5% (w/v), und Bentonit in einer Menge von 0,05 - 5,0% (w/v), vorzugsweise 0,1 - 2% (w/v), enthält.

**Revendications**

1. Suspension aqueuse de sucralfate, dans laquelle est ajouté de l'hydroxyéthylamidon ou de l'hydroxypropylamidon.

2. Suspension aqueuse selon la revendication 1, dans laquelle on ajoute en plus de la bentonite.

3. Suspension aqueuse selon la revendication 1 ou 2, qui comprend du sucralfate en une quantité de 0,1 - 50,0 p/v%, de préférence de 0,5 - 20,0 p/v%, de l'hydroxyéthylamidon ou de l'hydroxypropylamidon en une quantité de 0,05 - 10,0 p/v%, de préférence de 0,1 - 5,0 p/v%, et de la bentonite en une quantité de 0,05 - 5,0 p/v%, de préférence de 0,1 - 2,0 p/v%.